# FASCICULE DE BREVET EUROPEEN

(11) **EP 1 135 360 B1**
(45) Date de publication et mention de la délivrance du brevet: **31.03.2004**
(21) Numéro de dépôt: 99973015.3
(22) Date de dépôt: 01.12.1999
(51) Int. Cl.: C07C 217/58, A61K 31/135

(54) **NOUVEAUX DERIVES DE 3-ALKOXYBENZYLAMINES ET LEUR UTILISATION A TITRE DE MEDICAMENTS POUR LE TRAITEMENT DE LA SCHIZOPHRENIE**
3-ALKOXYLBENZYLAMINDERIVATE UND IHRE VERWENDUNG ALS ARZNEIMITTEL ZUR BEHANDLUNG VON SCHIZOPHRENIE
NOVEL 3-ALKOXYBENZYLAMINE DERIVATIVES AND THEIR USE AS MEDICINES FOR TREATING SCHIZOPHRENIA

(30) Priorité: 02.12.1998 FR 9815207
(43) Date de publication de la demande: 26.09.2001
(73) Titulaire: PIERRE FABRE MEDICAMENT, 92100 Boulogne-Billancourt (FR)
(72) Inventeur: Vacher, Bernard, 81100 Castres (FR); Cuisiat, Stéphane, 81100 Castres (FR); Koek, Wouter, 81290 Viviers-les-Montagnes (FR)
(74) Mandataire: Ahner, Francis
(86) Numéro de dépôt international: PCT/FR1999/002981
(87) Numéro de publication internationale: WO 2000/032557

(56) Documents cités:
- EP-A- 0 693 474
- EP-A- 0 707 007
- WO-A-98/08817
- FR-A- 2 702 211
- MEWSHAW R E ET AL: "New generation dopaminergic agents. 2. discovery of 3-OH -phenoxyethylamine and 3-OH-N-phenylpiperazine dopaminergic templates" BIOORGANIC & MEDICINAL CHEMISTRY LETTERS, vol. 8, no. 3, 3 février 1998 (1998-02-03), page 295-300 XP004136867 ISSN: 0960-894X cité dans la demande

## Description

La dopamine est un neuromédiateur qui participe au contrôle de la motricité, aux fonctions cognitives, à l'humeur, et intervient sur le circuit de récompense. Cinq types de récepteurs dopaminergiques ont été clonés (D₁-D₅), leurs niveaux d'expression et leurs distributions cérébrales ont été analysé. Parmi ces cinq types de récepteurs, deux types au moins possèdent des isoformes (Proc. Natl. Acad. Sci. USA 1998, 95, 7731). Ces cinq types de récepteurs dopaminergiques bien que pharmacologiquement distincts ont été regroupés en 2 sous-familles : la sous-famille D₁, qui comprend les récepteurs D₁ et D₅ et la sous-famille D₂ qui comprend les récepteurs D₂, D₃ et D₄. Il est possible de différencier l'action pharmacologique des sous-familles D₁ et D₂ mais il est difficile, en général, de différencier la fonction des différents types à l'intérieur de chaque sous-famille.

Un dysfonctionnement de la transmission dopaminergique est impliqué dans la symptomatologie des troubles du système nerveux central tels que les psychoses schizophréniques (Neuropsychopharmacol. 1988, 1, 179), certaines maladies neurodégénératives telle que, par exemple, la maladie de Parkinson (Neurodegenerative Diseases; Jolles, G. ; Stutzmann, J.M.; Eds; Academic Press, 1994, Chap. 8), la dépression (J. Clin. Psychiatry, 1998, 59 (Suppl. 5), 60), la dépendance à certaines substances telles que par exemple la cocaïne, le tabac ou l'alcool (Cell 1997, 90, 991; Nature 1997, 388, 586).

Ainsi, par exemple, les antagonistes des récepteurs dopaminergiques centraux du type D₂ constituent une approche classique et cliniquement efficace du traitement des symptômes positifs des psychoses schizophréniques. Cependant, la plupart des composés possédant un tel mécanisme d'action induisent aussi des effets secondaires indésirables tels que des symptômes de type Parkinsonien (Pharmacotherapy 1996, 16, 160) et / ou des désordres neuroendocriniens (Acta Psychiatr. Scand. 1989, 352, 24).

Mewshaw et col. (Bioorg. Med. Chem. Lett. 1998, 8, 295) décrit des phenoxyéthylamines de formule : 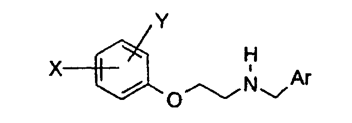 où X représente un atome d'hydrogène, un groupe hydroxyle, un groupe amino ou un groupe méthanesulfonamide, Y représente un atome d'hydrogène ou un atome d'halogène et Ar est un groupe phényl ou 2-thiényl, comme étant des agonistes partiels des récepteurs du type D₂.

Les brevets WO 9808817, US 5760070, WO 9808843 et WO 9808819 décrivent respectivement des 4-aminoéthoxyindoles et des 4-aminoéthoxyindolones comme étant des agonistes des récepteurs dopaminergiques du type D₂ ou des inhibiteurs de la synthèse et de la libération de la dopamine.

Unangst et Col. (J. Med. Chem. 1997, 40, 4026) décrit des aryloxyalkylamines de formule : 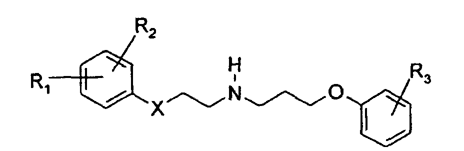 où X représente un atome d'oxygène, de soufre ou un groupe CH₂; R₁ est un atome d'hydrogène, de chlore, un groupe hydroxyle ou hydroxyméthyle, un groupe nitro ou un résidu hydroxycarbonyle; R₂ et R₃ représentent un atome d'hydrogène, un atome d'halogène ou un groupe méthyle. Ces composés sont actifs sur le système dopaminergique, en particulier sur les récepteurs du type D₄ et potentiellement utiles dans le traitement de la schizophrénie.

Le brevet WO 9723482 décrit des octahydropyrrolo[1,2-a]pyrazines de formule : 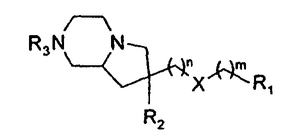 où X représente, entre autres, un atome d'oxygène; m et n = 0, 1, 2 et R₁ est un groupe aromatique non substitué, hétérocyclique ou non, polycyclique ou non. Ces composés ont une affinité pour les récepteurs dopaminergiques, en particulier pour les récepteurs du type D₄.

Les brevets FR 2.702.211, JP 51048627, JP 51052146, DE 2450616 et WO 9631461 décrivent des dérivés de 2-[2-(alkoxy)phénoxy]éthylamines de formule : 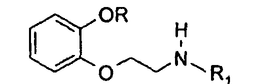 dans laquelle R est un groupe alkyl en C₁-C₄ et R₁ représente une chaîne 4-benzenebutyl, pipéridine-4-méthyl ou 4-benzamidobutyl. Ces composés sont revendiqués comme étant des ligands des récepteurs du sous type 5-HT_{1A} (FR.2.702.211 et WO 9631461) ou des agents hypotenseurs et tranquillisants (JP 51048627, JP 51052146 et DE 2450616).

Le brevet EP 707007 décrit des arylamines présentant une double activité : à la fois antagoniste des récepteurs du type D₂ et agoniste des récepteurs du sous type 5-HT_{1A} et utiles comme agents antipsychotiques. Le composé EMD-12830 (Drug Data Report 1998, 21) de formule : 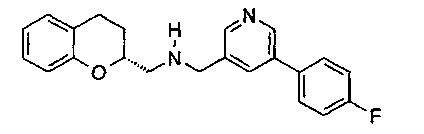 est revendiqué comme un agent antipsychotique atypique (i.e., présentant une moindre propension à provoquer des effets secondaires de type parkinsonien que les antipsychotiques conventionnels).

Le brevet DE 2364685 décrit des phénoxyalkylamines, en particulier la N-[2-(2-méthoxyphénoxy)éthyl]-pyridin-3 ou 4-ylméthanamine sont revendiquées comme des agents hypotenseurs.

Angstein et col. (J. Med. Chem. 1965, 8, 356) décrit des aryloxyalkylamines actives sur le système cardio-vasculaire. Parmi les composés décrits figure la N-[2-(2-méthoxyphénoxy)éthyl]-benzèneméthanamine.

Goldenberg et Col. (Chim. Ther. 1973, 8, 259) décrit, entre autres, des N-[2-(2-méthoxyphénoxy)éthyl-2-benzofuraneméthanamines comme des agents ayant des propriétés vasodilatatrices périphériques.

Le 4-méthoxy-3-[2-[(phénylméthyl)amino]éthoxy]-phénol est décrit dans J. Labelled Compd. Radiopharm. 1993, 33, 1091 et la N-[2-(2-méthoxyphenoxy)éthyl]-furfurylamine est décrite dans Fr. 1.336.684.

Des dérivés de 3-(cyclopentyloxy)-benzèneméthanamine de formule : 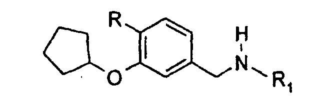 où R représente un groupe hydroxy ou méthoxy et R₁ représente un groupe aromatique, un groupe acyle ou thioacyle substitué, sont revendiqués comme inhibiteurs de phosphophodiestérases dans les brevets WO 9746561, WO 9520578, WO 9504045, WO 9402465 et WO 9315044 ou comme agents actifs dans le traitement de l'insuffisance cardiaque dans le brevet US 4971959.

Le brevet WO9200968 décrit des dérivés de formule: 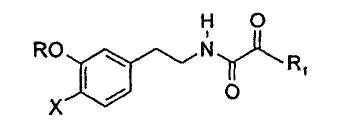 où R représente un groupe cycloalkyle en C₄-C₆ et X est un atome d'hydrogène, de fluor ou de chlore, sont revendiqués pour leur activité dans le contrôle des désordres dépendants de la production de TNF.

Le brevet WO 9801417 décrit des composés aromatiques et hétéroaromatiques de formule : 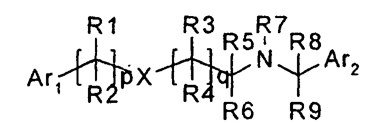 ou Ar₂ est, entre autres, un groupe aromatique éventuellement substitué par un groupe alkoxy inférieur non cyclique. Ces composés sont revendiqués comme influençant les récepteurs du calcium.

### Résumé de l'invention

La présente invention concerne une nouvelle famille de composés qui répondent à la formule générale (1)

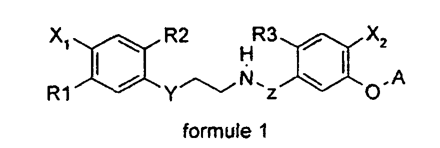

Les composés de cette invention ont une activité antidopaminergique en particulier sur les récepteurs de la sous-famille D₂. A ce titre, les composés de l'invention sont utiles dans le traitement des affections résultant d'une hyperactivité dopaminergique tels que les symptômes schizophréniques et la dépendance à certaines substances. Cependant, l'activité antagoniste des produits de l'invention sur les récepteurs du type D₂, ne s'exerce que lors d'une hyperstimulation dopaminergique transitoire. En l'absence d'une hyperactivité dopaminergique, c'est-à-dire lorsque la concentration en dopamine varie dans des proportions acceptables pour le fonctionnement normal du neurone, les composés de l'invention n'induisent pas une hypoactivité dopaminergique. Les composés de l'invention sont donc utiles dans le traitement des symptômes schizophréniques et présentent l'avantage d'être potentiellement dépourvus des effets secondaires indésirables occasionnés par un blocage excessif des récepteurs du type D₂, tels que les symptômes Parkinsonien et / ou les désordres endocriniens, aux doses thérapeutiquement efficaces pour le traitement des psychoses schizophréniques.

Les composés de l'invention diffèrent donc des dérivés de l'art antérieur par leur formule chimique et leur mécanisme d'action.

### Détails de l'invention

Plus spécifiquement, la présente invention concerne des composés nouveaux répondant à la formule générale (1). 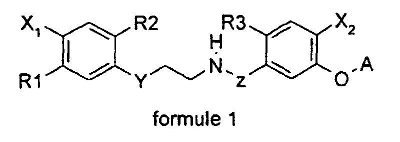 dans laquelle :
X₁ est un atome d'hydrogène, de chlore ou de fluor ;
X₂ à la même signification que X₁ ;
R₁ représente :
   - un atome d'hydrogène, de chlore, ou de fluor ;
   - un substituant R₄, un groupe hydroxy (OH), un groupe alkoxy (OR₄), un groupe alkylcarbonyloxy (OC(O)R₄), un groupe alkylcarbonyl (C(O)R₄), un groupe amino (NH₂), un groupe alkylamino (NHR₄), un groupe dialkylamino (N(R₄)₂), un groupe (NHC(O)R₄) ou un groupe cyano (CN) ;
R₂ est un substituant R₄ ou un groupe alkoxy (OR₄) ;
R₃ à la même signification que R₁ ;
R₄ représente :
   - un radical alkyle en C₁-C₅ linéaire ou ramifié, substitué ou non par 1 ou 2 atomes de fluor ou un groupe hydroxy (OH), contenant ou non une double liaison ;
   - un radical cycloalkyle à 3, 4, 5 ou 6 chaînons, substitué ou non par 1 ou 2 atomes de fluor, contenant ou non une double liaison ;
Y est un atome d'oxygène, un groupe fluorométhylene (CHF) ou difluorométhylene (CF₂) ;
z est un groupe méthylene (CH₂), éventuellement substitué par 1 ou 2 groupes méthyl (CH₃) ou fluorométhyl (CH₂F) ;
A représente :
   - un radical cycloalkyle à 3, 4, 5 ou 6 chaînons ou un radical bicyclique à 7 ou 8 chaînons, contenant éventuellement une double liaison, une fonction oxo (=O), un groupe hydroxy (OH), un groupe méthoxy (OCH₃) ou 1 ou 2 atomes de fluor ;
   - un groupe hétérocyclique non aromatique à 5 ou 6 chaînons contenant un ou deux hétéroatomes choisis parmi l'azote, l'oxygène, ou le soufre, éventuellement substitué par une fonction oxo (=O), un groupe hydroxy (OH), un groupe méthoxy (OCH₃) ou 1 ou 2 atomes d'halogène.

L'invention concerne également les sels d'addition et éventuellement les hydrates des sels d'addition des composés de formule générale (1) avec les acides minéraux ou les acides organiques pharmaceutiquement acceptables.

L'invention a aussi pour objet les compositions pharmaceutiques contenant à titre de principe actif au moins un des dérivés de formule générale (1) ou un de ses sels ou hydrates de ses sels en combinaison avec un ou plusieurs excipients, adjuvants ou véhicules pharmaceutiquement acceptables. A titre d'exemple on peut citer les complexes d'inclusion, en particulier les complexes d'inclusion formés par les composés de l'invention avec les β-cyclodextrines.

Les compositions pharmaceutiques selon l'invention peuvent être des compositions administrables par voie orale, nasale, sublinguale, rectale ou parentérale. Il est généralement avantageux de formuler de telles compositions pharmaceutiques sous forme de dose unitaire. Chaque dose comprend alors une quantité prédéterminée du principe actif, associée au véhicule, excipients et / ou adjuvants appropriés, calculés pour obtenir un effet thérapeutique donné. A titre d'exemple de forme de dose unitaire administrable par voie orale on peut citer les comprimés, les gélules, les granules, les poudres et les solutions ou suspensions orales.

Les formulations appropriées pour la forme d'administration choisie sont connues et décrites, par exemple dans : Remington, The Science and Practice of Pharmacy, 19^{ième} edition, 1995, Mack Publishing Company et peuvent donc être facilement préparées par l'homme de l'art.

Il est connu que la posologie varie d'un individu à l'autre, selon la nature et l'intensité de l'affection, la voie d'administration choisie, le poids, l'âge et le sexe du malade en conséquence les doses efficaces devront être déterminées en fonction de ces paramètres par le spécialiste en la matière. A titre indicatif, les doses efficaces pourraient s'échelonner entre 0.001 et 100 mg/Kg/jour.

Les composés de formule générale (1) peuvent exister sous plusieurs formes tautomères. De telles formes tautomères quoique non explicitement rapportées dans la présente demande pour simplifier la représentation graphique des formules développées sont néanmoins incluses dans le champ d'application de l'invention.

Lorsque les composés de l'invention comportent un atome de carbone asymétrique, l'invention concerne aussi bien les mélanges racémiques que les différents énantiomères du composé en question ainsi que leurs mélanges en toutes proportions.

Selon une caractéristique particulière de la présente invention, R₂ représente un isopropoxy.

Selon une autre caractéristique particulière de la présente invention, Y représente un atome d'oxygène et Z représente un groupe de méthylène.

Selon une autre caractéristique particulière de la présente invention, A est choisi parmi les groupes cyclopentyle, cyclohexyle, cyclohex-2-ényle, bicyclo[2.2.1 ]hept-5-èn-2-yle.

Les composés de formule générale dans lesquels :
- Y à la même signification que précédemment,
- z représente un radical méthylène substitué ou non par un groupe méthyle ou fluorométhyle,
- X₁, X₂, R₁, R₂, R₃, R₄ et A ont la même signification que précédemment,
peuvent être préparés selon le procédé décrit dans le schéma A.

### Schéma A

Le composé de formule (1) est préparé par une réaction classique d'amination réductrice entre le composé de formule (2), dans lequel w représente un atome d'hydrogène, un radical méthyle ou fluorométhyle, et l'amine primaire de formule (3). L'expression "une réaction classique d'amination réductrice" signifie que le composé de formule (2) et l'amine (3) sont mis en réaction dans le solvant approprié et que le mélange des réactifs (2) et (3) est ensuite soumis à l'agent réducteur selon une méthode bien connue de l'homme de l'art.

Les composés de formule (1) sont purifiés suivant une ou plusieurs méthodes choisies parmi la cristallisation et / ou les techniques de chromatographie en phase liquide. Ils peuvent être ensuite, si on le désire :
- salifiés au moyen d'un acide pharmaceutiquement acceptable ;
- engagé dans la formation d'un complexe d'inclusion.

Le procédé de préparation des amines primaires de formule (3) dépend de la nature des substituants X₁, R₁ et R₂ portés par le noyau benzénique.

Le dérivé de formule (4a), précurseur de l'amine primaire (3), dans lequel :
- X₁ est un atome d'hydrogène, de chlore ou de fluor,
- R₁ est un atome d'hydrogène, de fluor, un groupe formyle, un groupe nitro ou un groupe éthoxycarbonyle (C(O)OEt),
peut-être obtenu par le procédé décrit dans le schéma B.

### Schéma B

Le 4-nitrocatéchol est régiosélectivement alkylé au moyen d'un 2-halogenopropane pour donner le dérivé (5-3 ; R₁ = NO₂), selon un protocole expérimental analogue à celui décrit dans (Org. Prep. Proced. Int. 1992, 23, 753). Le 2-hydroxy-3-isopropoxy-benzaldehyde (5-2 ; R₁ = CHO) est un composé connu dans la littérature chimique (Hétérocycles 1984, 22 (9), 1995). La préparation du 2-isopropoxy-5-éthoxycarbonyl-phénol (5-1 ; R₁ = CO₂Et) est décrite dans EP 579223. Une réaction classique de Williamson (J. Med. Chem. 1989, 32, 105) entre les composés de formule (5) appropriés et le 1-bromo-2-chloro-éthane conduit à l'éther chloré correspondant, qui par réaction avec du phtalimide de potassium (synthèse de Gabriel) donne les amines protégées de formule (4a) dans lesquelles R₁ est H, CHO, NO₂, CO₂Et et X₁ est un atome d'hydrogène. Dans des conditions similaires à celles utilisées pour la transformation du phénol de formule (5) en l'amine protégée de formule (4a), la 5-fluoro-2-hydroxy-acétophénone est convertie en l'amine protégée de formule (7 ; R = CH₂CH₂NPht). Une réaction de Bayer-Villiger effectuée sur le composé de formule (7), Synth. Commun 1989, 11/12, 2001, suivie d'une réaction d'hydrolyse basique du formate intermédiaire conduit au phénol de formule (6 ; R = CH₂CH₂NPht). L'alkylation du phénol de formule (6), dans les conditions usuelles, donne le composé de formule (4a) dans lequel R₁ est un atome de fluor ou de chlore et X₁ est un atome d'hydrogène. L'invertion de l'ordre d'incorporation des résidus alkyles au niveau des intermédiaires de formules (7) et (6) permet d'accéder, dans des conditions expérimentales identiques à celles décrites précédemment, aux composés de formule (4a) dans lesquels R₁ est un atome d'hydrogène et X₁ est un atome de fluor ou de chlore.

Les amines dérivés de formule (4b-f), précurseurs des amine primaires (3), dans lesquelles :
- X₁ est un atome d'hydrogène,
- R₁ est un groupe R₄, C(O)R₄, OH ou OR₄,
peuvent être obtenues selon le procédé décrit dans le schéma C.

### Schéma C

Le composé de formule (4a-2) est l'intermédiaire utilisé pour la préparation des amines protégées de formules (4b-g).

Selon la voie a : une réaction de Wittig classique entre l'intermédiaire de formule (4a-2) et l'iodure de méthyltriphényl phosphonium conduit au dérivé vinylique de formule (4b). Le dérivé de formule (4b) peut :
- soit être engagé directement dans la préparation des composés de formule (1) dans lesquels R₄ est un groupe vinyle ;
   - soit être réduit, dans des conditions classiques d'hydrogénation catalysée par les métaux de transition, pour donner le composé de formule (4c) ;
   - soit être oxydé, selon une réaction de Wacker classique (Org. Synth. 1988, 67, 12) pour donner le composé de formule (4d). Un procédé analogue au procédé décrit par la voie a peut alors être réitéré à partir de la cétone (4d) pour donner le composé de formule (4e).

L'utilisation d'un ylure de phosphonium non stabilisé, dérivé d'un halogénure d'alcane supérieur, permet d'accéder aux dérivés de formule (4b-e) où R₁ représente un groupe R₄ ou C(O)R₄ différent d'un groupe méthyle.

La fonction cétone du dérivé de formule (4d) peut être également réduite en une fonction alcool secondaire tel que, par exemple, celle présente dans le composé 4h.

Selon la voie b : une réaction de Bayer-Villiger, dans des conditions identiques à celles décrites pour la préparation du phénol de formule (6) à partir de l'intermédiaire de formule (7), Schéma B, conduit au phénol de formule (4f) qui peut-être ensuite alkylé, dans les conditions usuelles, pour donner les dérivés de formule (4g).

Les dérivés de formule (4i), précurseurs des amines primaires (3), dans lesquelles :
- X₁ est un atome d'hydrogène,
- R₁ est une groupe NH₂, NHR₄, N(R₄)₂ ou NHC(O)R₄, sont obtenus selon le procédé décrit dans le schéma D.

### Schéma D

L'intermédiaire de formule (4a-6) est préparé au moyen d'une réaction de Mitsunobu entre le composé de formule (5 ; R₁ = NO₂, Schéma A) et le (2-hydroxy-éthyl)-carbamic acid tert-butyl ester (Eur. J. Med. Chem. 1995, 30, 387). La réduction de la fonction nitro du composé de formule (4a-6), J. Org. Chem. 1987, 52, 1844, conduit à l'amine de formule (4g-2). L'acylation de l'amine de formule (4g-2), dans des conditions expérimentales classiques, conduit au dérivé de formule (4i) dans lequel R₁ est un groupe (R₄CONH). Les dérivés de formule (4i) dans lesquels R₁ représente un groupe NHR₄ ou N(R₄)₂ sont facilement préparés à partir du composé de formule (4g-2) au moyen de réactions bien connues de l'homme de l'art.

Les composés de formule (4j) ou (4k) dans lesquels : 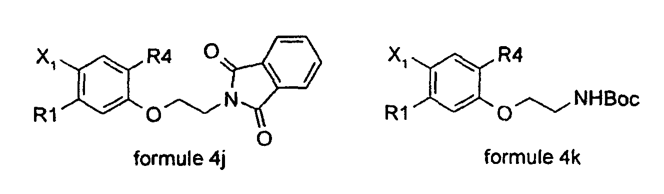 X₁, R₁ et R₄ ont la même signification que précédemment sont préparés selon des procédés analogues à ceux décrits pour la préparation des composés de formule (4a-i) à partir des phénols convenablement substitués, disponibles commercialement ou préparés selon des méthodes connues dans la littérature chimique.

Les amines primaires de formule (3), préparées par déprotection des composés de formule (4a-i), sont utilisées immédiatement dans l'étape suivante d'amination réductrice (Schéma A). Les méthodes de déprotection des amines de formules (4a-i) sont décrites dans le schéma E.

### Schéma E

La déprotection des composés de formule (4a-h) est effectué par chauffage modéré (60°C) des dérivés de formule (4a-h) en présence d'un excès d'amino-2-ethanol (voie a). La déprotection des composés de formule (4i) est effectuée par traitement d'une solution de composés (4i) dans le dichlorométhane au moyen d'un excès d'acide trifluoroacétique (voie b).

Le procédé de préparation des aldéhydes de formule (2), Schéma A, dépend de la nature du substituent R₃.

Les aldéhydes de formule (2a) dans lesquels :
- R₃ est un atome d'hydrogène ou un groupe OCH₃,
- A à la même signification que précédemment,
sont préparés par le procédé décrit dans le schéma F.

### Schéma F

Le 3-hydroxy-benzaldéhyde ou le 2-méthoxy-5-hydroxy-benzaldéhyde, préparé selon J. Org. Chem. 1974, 39, 2437, est alkylé au moyen de l'halogénure de cycloalcane, ou de cycloalcène approprié, dans des conditions identiques à celles décrites précédemment pour la formation de l'éther de formule (5) à partir du 4-nitro-catéchol (Schéma B), pour donner les composés de formule (2a) dans lesquels R₃ est un atome d'hydrogène ou un groupe méthoxy. Cependant, lorsque A représente un groupe bicyclique, par exemple un groupe. bicyclo[2.2.1]hept-5-ene, la réaction d'étherification en question est accomplie de préférence à partir de l'alcool bicyclique approprié au moyen d'une réaction de Mitsunobu selon une procédure identique à celle décrite dans (J. Med. Chem. 1991, 34, 291).

Les aldéhydes de formules (2b) dans lesquels :
- R₃ est un atome de fluor ou un atome de chlore,
- A à la même signification que précédemment,
sont préparés par le procédé décrit dans le schéma G.

### Schéma G

Le 3-méthyl-4-halogeno-phénol approprié (R₃ = Cl ou F) est alkylé dans des conditions identiques à celles utilisées pour la préparation du composé de formule (2a) à partir du phénol correspondant (schéma F). Le composé de formule (8) est ensuite bromé sélectivement sur le groupement méthyle en position benzylique (J. Med. Chem. 1982, 25, 1204) pour donner le composé de formule (9) qui est oxydé en l'aldéhyde de formule (2b) selon la méthode développée par Komblum (J. Org. Chem. 1986, 51, 1264).

Les aldéhydes de formule (2c) dans lesquels :
- X₂ est un atome de fluor ou de chlore,
- A à la même signification que précédemment,
peuvent être préparés selon une méthode identique à celle décrite dans WO 9200968.

Les composés de formule (4a-i) et (2a-c) constituent l'ensemble des composés de formule (4) et (2).

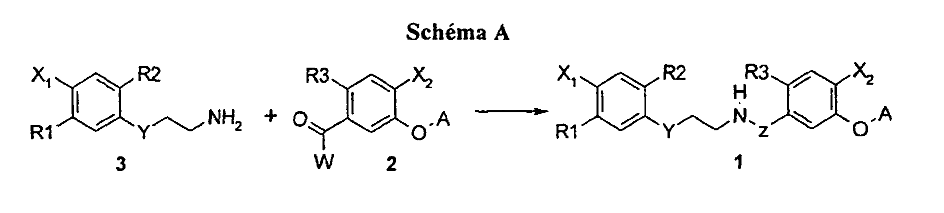

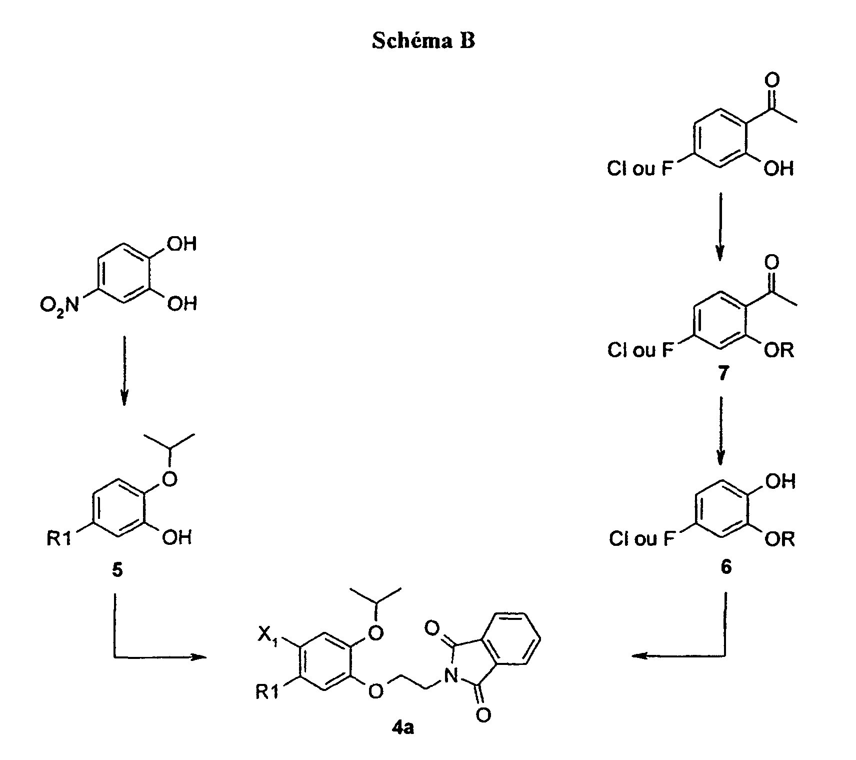

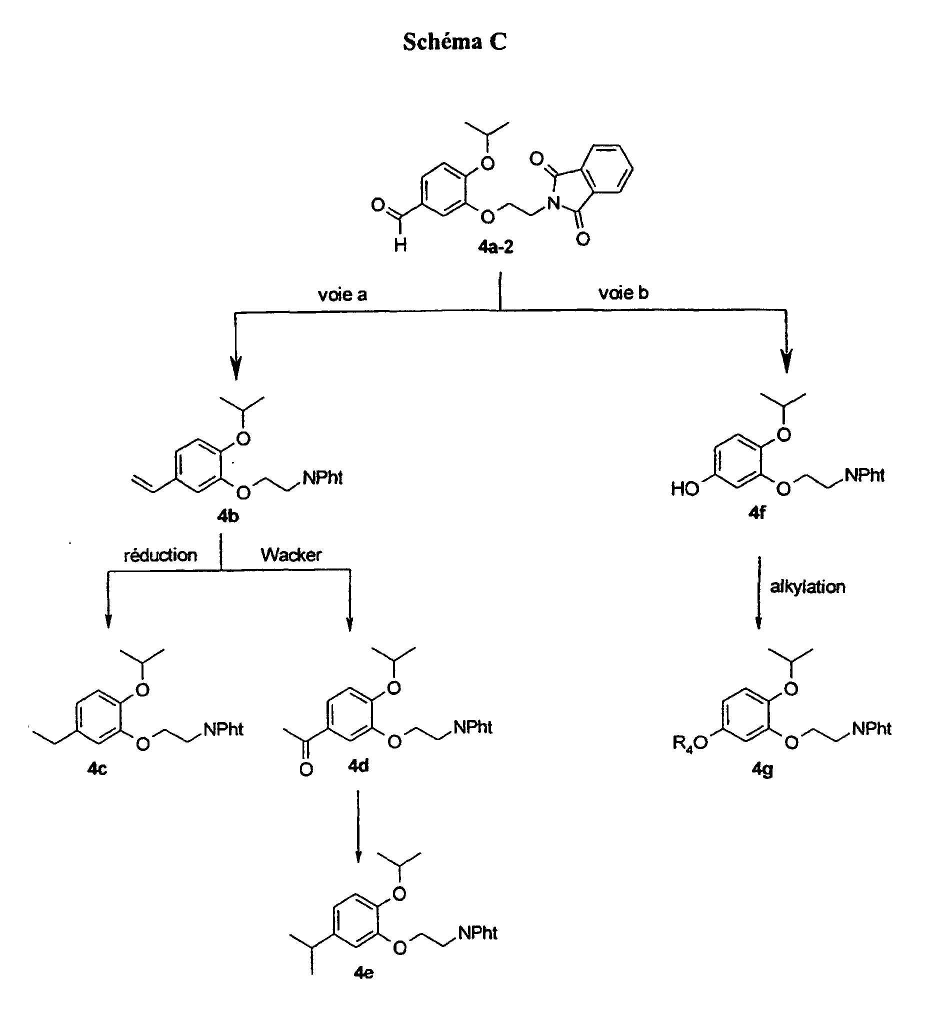

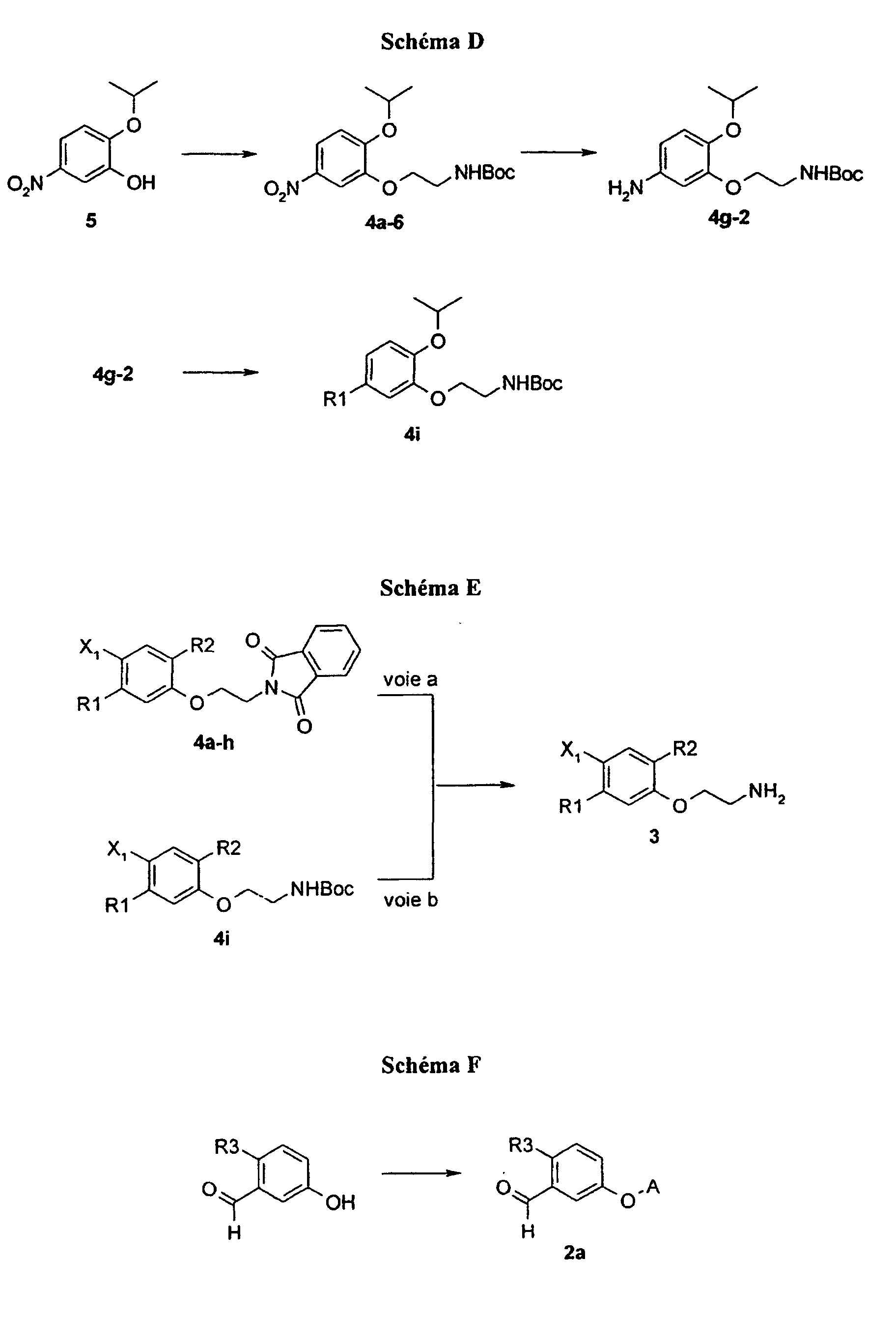

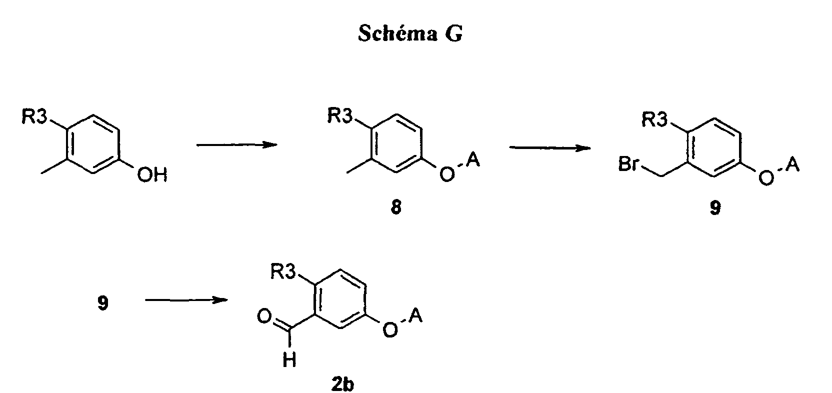

Les exemples suivants illustrent l'invention sans toutefois en limiter la portée. Dans les exemples ci-après :
(i) l'avancement des réactions est suivi par chromatographie couche mince (CCM) et par conséquent les temps de réaction ne sont mentionnés qu'à titre indicatif.
(ii) des formes cristallines différentes peuvent donner des points de fusions différents, les points de fusion rapportés dans la présente demande sont ceux des produits préparés selon la méthode décrite et ne sont pas corrigés.
(iii) la structure des produits obtenus selon l'invention est confirmée par les spectres de résonance magnétique nucléaire (RMN), infrarouge (IR) et l'analyse centésimale, la pureté des produits finaux est vérifiée par CCM.
(iv) les spectres RMN sont enregistrés dans le solvant indiqué. Les déplacements chimiques (δ) sont exprimés en partie par million (ppm) par rapport au tétraméthylsilane. La multiplicité des signaux est indiquée par : s, singulet ; d, doublet ; t, triplet ; q, quadruplet ; m, multiplet ; 1, large.
(v) les différents symboles des unités ont leur signification habituelle : mg (milligramme) ; g (gramme) ; ml (millilitre) ; °C (degré Celsius) ; mmole (millimole) ; nmole (nanomole) ; cm (centimètre).
(vi) Les abréviations ont la signification suivante : F (point de fusion) ; Eb (point d'ébullition).
(vii) Dans la présente application les pressions sont données en millibars ; par "température ambiante" on entend une température comprise entre 20°C et 25°C.

### Exemple 1 : 2-isopropoxy-5-nitrophénol (5)

Une solution de 15.5 g de 4-nitrocatéchol (100 mmoles) dans 200 ml de N,N-diméthylformamide est ajoutée goutte à goutte à 0°C à une suspension d'hydrure de sodium 60% dans l'huile, 4.2 g (105 mmoles). A la fin de l'addition (1 heure) la solution rouge foncée est agitée à 0°C pendant 1.5 heures, puis 10.5 ml de 2-iodopropane (105 mmoles) sont ajoutés goutte à goutte. Le mélange est ensuite porté à 80°C et agité pendant 16 heures. Il est ensuite refroidi à température ambiante, le solvant est évaporé et l'huile rouge foncée est dissoute dans du dichlorométhane (300 ml). La solution est lavée à l'eau puis avec une solution aqueuse saturée de chlorure de sodium, la phase organique est séchée sur sulfate de sodium, filtrée et le solvant est évaporé. Le produit attendu est isolé par chromatographie sur colonne de silice (éluant : cyclohexane / acétate d'éthyle, 80 : 20). On récupère 10.5 g d'une huile jaune qui se solidifie :
Rendement : 53%
F : 139°C
¹H RMN (DMSO d₆) δ : 1.31 (d, 6H); 4.76 (m, 1H); 7.24 (d, 1H); 7.63 (d, 1H); 7.76 (dd, 1H).

### Exemple 2 : 2-[2-(2-acétyl-5-fluoro-phénoxy)-éthyl]-isoindole-1,3-dione (7)

### Etape 1 : 1-[2-(2-chloro-éthoxy)-4-fluoro-phényl]-éthanone

On ajoute à température ambiante 40.5 ml de 1-bromo-2-chloroethane (490 mmoles) à une solution de 25 g de 1-[2-hydroxy-4-fluoro-phényl]-éthanone (162 mmoles) dans la 2-butanone (400 ml), suivi de 45 g de carbonate de potassium (320 mmoles) et de 1.26 g d'iodure de potassium (7.59 mmoles).

Le mélange est chauffé à 80°C sous agitation vigoureuse pendant 60 heures, puis est refroidi à température ambiante et versé dans de l'eau glacée. On extrait par de l'acétate d'éthyle et on lave la phase organique par une solution aqueuse saturée de chlorure de sodium. La phase organique est ensuite séchée sur sulfate de magnésium, filtrée et le solvant évaporé. Le produit est cristallisé dans un mélange cyclohexane / acétate d'éthyle. On récupère 15.7 g d'un solide blanc :
Rendement : 45%
F : 67°C
¹H RMN (CDCl₃) δ : 2.66 (s, 3H) ; 3.91 (t, 2H) ; 4.32 (t, 2H) ; 6.62 (dd, 1H) ; 6.76 (dt, 1H) ; 7.85 (dt, 1H).

### Etape 2 : 2-[2-(2-acétyl-5-fluoro-phénoxy)-éthyl]-isoindole-1,3-dione

Un mélange de 14.6 g de phtalimide de potassium (79 mmoles) et de 15 g de 1-[2-(2-chloro-éthoxy)-4-fluoro-phényl]-éthanone (69.2 mmoles) dans 150 ml de N,N-diméthylformamide est chauffé à 150°C pendant 6 heures. Le mélange est ensuite refroidi et le solvant est évaporé sous vide. Le solide obtenu est repris dans du dichlorométhane, la phase organique est lavée à l'eau puis avec une solution aqueuse saturée de chlorure de sodium. La phase organique est ensuite séchée sur du sulfate de magnésium, filtrée et le solvant est évaporé. Le produit obtenu est purifié par cristallisation dans un mélange cyclohexane / acétate d'éthyle. On obtient 18.2 g d'un solide blanc :
Rendement : 76%
F : 140°C
¹H RMN (CDCl₃) δ : 2.55 (s, 3H) ; 4.19 (t, 2H) ; 4.34 (t, 2H) ; 6.67 (m, 2H) ; 6.69 (m, 1H) ; 7.77 (m, 2H) ; 7.80 (m, 2H).
IR (KBr) ν : 1774, 1716, 1679, 1605, et 1590 cm⁻¹.

### Exemple 3 : 2-[2-(5-fluoro-2-hydroxy-phénoxy)-éthyl]-isoindole-1,3-dione (6)

Une solution de 26 g d'acide métachloroperbenzoïque (à 55%, 82.9 mmoles) dans 220 ml de dichlorométhane est agitée pendant une heure puis transférée dans une ampoule de décantation. La phase aqueuse est séparée, la phase organique est placée dans un ballon et refroidie à 0°C. 18 g de 2-[2-(2-acetyl-5-fluoro-phenoxy)-éthyl]-isoindole-1,3-dione (55 mmoles) sont ajoutés par portions et le mélange est agité à température ambiante pendant 16 heures. 6.9 g de bicarbonate de sodium (82 mmoles) sont ensuite introduit par portion et le mélange est agité pendant une heure. Le mélange est ensuite concentré sous vide, 200 ml de méthanol sont ajoutés suivi de 15.2 g de carbonate de potassium (110 mmoles). Le mélange est agité pendant 4 heures à température ambiante, puis le solvant est évaporé, remplacé par 200 ml de dichlorométhane et le mélange est lavé à l'eau et avec une solution aqueuse saturée de chlorure de sodium. La phase aqueuse est séchée sur sulfate de sodium, filtrée et le solvant évaporé. Le produit est cristallisé dans du dichlorométhane. On obtient 14 g de produit du titre sous la forme d'un solide blanc :
Rendement : 84%
F : 172°C
¹H RMN (DMSO d₆) δ : 3.95 (t, 2H) ; 4.22 (t, 2H) ; 6.58 (dt, 1H) ; 6.75 (t, 1H) ; 6.85 (m, 1H) ; 7.64 (m, 4H) ; 8.77 (s, 1H (échangeable)).

### Exemple 4 : 2-[2-(2-isopropoxy-phénoxy)-éthyl]-isoindole-1,3-dione (4a-1)

En procédant comme dans l'exemple 2 mais en remplaçant dans l'étape 1 le 1-[2-hydroxy-4-fluorophényl]-éthanone par le 2-isopropoxy-phénol :
Rendement global: 96%
F : 72°C
¹H RMN (CDCl₃) δ : 1.24 (d, 6H) ; 4.13 (t, 2H) ; 4.26 (t, 2H) ; 4.40 (m, 1H) ; 6.88 (m, 4H) ; 7.72 (m, 2H) ; 7.84 (m, 2H).

### Exemple 5 : 3-[2-(1,3-dioxo-1,3-dihydro-isoindol-2-yl)-éthoxy]-4-isopropoxy-benzaldéhyde (4a-2)

En procédant comme dans l'exemple 2 mais en remplaçant dans l'étape 1 la 1-[2-hydroxy-4-fluorophényl]-éthanone par le 3-hydroxy-4-isopropoxy-benzaldehyde on prépare le composé du titre sous forme de poudre blanche :
Rendement global : 95%
F : 110°C

| Analyse C₂₀H₁₉NO₅ | | | |
|---|---|---|---|
| Calc % | C 67.98 | H 5.42 | N 3.96 |
| Tr. | 67.55 | 5.30 | 4.29 |

¹H RMN (CDCl₃) δ : 1.28 (d, 6H) ; 4.19 (t, 2H) ; 4.32 (t, 2H) ; 4.54 (m, 1H) ; 6.91 (d, 1H) ; 7.43 (m, 2H) ; 7.74 (m, 2H) ; 7.84 (m, 2H) ; 9.83 (s, 1H).

### Exemple 6 : 2-[2-(5-nitro-2-isopropoxy-phénoxy)-éthyl]-isoindole-1,3-dione (4a-3)

En procédant comme dans l'exemple 2 mais en remplaçant dans l'étape 1 la 1-[2-hydroxy-4-fluoro-phényl]-éthanone par le 2-isopropoxy-4-nitro-phenol on prépare le composé du titre sous forme d'une huile jaune qui cristallise :
Rendement global : 85%
F : 132°C
¹H RMN (DMSO d₆) δ : 1.12 (d, 6H) ; 4.02 (t, 2H) ; 4.37 (t, 2H) ; 4.64 (m, 1H) ; 7.12 (d, 1H) ; 7.77 (d, 1H) ; 7.86 (m, 5H).

### Exemple 7 : 2-[2-(5-Fluoro-2-isopropoxy-phénoxy)-éthyl]-isoindole-1,3-dione (4a-4)

En procédant comme dans l'exemple 1 mais en remplaçant le 2-isopropoxy-5-nitro-phénol par le 2-[2-(5-fluoro-2-hydroxy-phénoxy)-éthyl]-isoindole-1,3-dione on obtient le composé du titre sous forme d'un solide blanc :
Rendement : 77%
F : 68°C
¹H RMN (CDCl₃) δ : 1.20 (d, 6H) ; 4.14 (t, 2H) ; 4.23 (t, 2H) ; 4.30 (m, 1H) ; 6.56 (dt, 1H) ; 6.65 (dd, 1H) ; 6.79 (dd, 1H); 7.73 (m, 2H) ; 7.86 (m, 2H).

### Exemple 8 : 2-[2-(isopropoxy-5-vinyl-phénoxy)-éthyl]-isoindole-1,3-dione (4b)

On ajoute par fractions 0.42 g de tertiobutylate de potassium (3.75 mmoles) à une suspension de 1.34 g de bromure de méthyltriphénylphosphonium (3.75 mmoles) dans 7.5 ml de tétrahydrofurane. On agite le mélange pendant une heure à température ambiante, puis on refroidit le mélange à 0°C. On introduit ensuite une solution de 1.20 g (3.4 mmoles) de 3-[2-(1,3-dioxo-1,3-dihydro-isoindol-2-yl)-éthoxy]-4-isopropoxy-benzaldéhyde dans 7 ml de tétrahydrofurane. On agite alors le mélange à température ambiante pendant 16 heures, puis on le verse dans une solution aqueuse saturée en chlorure d'ammonium. On extrait au dichlorométhane, on lave la phase organique par une solution aqueuse saturée de chlorure de sodium, on sèche sur sulfate de magnésium, on filtre et le solvant est évaporé sous vide. Le résidu est purifié par chromatographie sur colonne de silice (éluant : cyclohexane / acétate d'éthyle ; 80 / 20). On obtient 0.77 g d'une huile jaune qui cristallise :
Rendement : 64%
F : 78°C
¹H RMN (CDCl₃) δ : 1.25 (d, 6H) ; 4.14 (t, 2H) ; 4.28 (t, 2H) ; 4.42 (m, 1H) ; 5.13 (d, 1H) ; 5.58 (d, 1H) ; 6.63 (dd, 1H) ; 6.89 (d, 1H) ; 6.92 (dd, 1H) ; 7.01 (d, 1H) ; 7.73 (m, 2H) ; 7.84 (m, 2H).

### Exemple 9 : 2-[2-(5-Éthyl-2-isopropoxy-phénoxy)-éthyl]-isoindole-1,3-dione (4c)

Dans une solution de 0.53 g de 2-[2-(isopropoxy-5-vinyl-phénoxy)-éthyl]-isoindole-1,3-dione (1.51 mmoles) dans 8 ml de méthanol, on ajoute 0.15 g de palladium sur charbon à 10%. La suspension est agitée sous atmosphère d'hydrogène pendant 2 heures, puis on élimine le solide par filtration sur filtre de silice et on évapore le solvant. Le produit du titre est isolé par cristallisation dans un mélange cyclohexane / acétate d'éthyle. On obtient 0.53 g d'un solide blanc :
Rendement : 100%
F:61°C
¹H RMN (CDCl₃) δ : 1.20 (t, 3H) ; 1.22 (d, 6H) ; 2.56 (q, 2H) ; 4.22 (t, 2H) ; 4.26 (t, 2H) ; 4.33 (m, 1H) ; 6.71 (dd, 1H) ; 6.76 (m, 2H) ; 7.74 (m, 2H) ; 7.83 (m, 2H).

### Exemple 10 : 2-[2-(5-Hydroxy-2-isopropoxy-phénoxy)-éthyl]-isoindole-1,3-dione (4f)

En procédant comme dans l'exemple 3, mais en remplaçant le 2-[2-(acétyl-5-fluoro-phénoxy)-éthyl]-isoindole-1,3-dione par le 3-[2-(1,3-dioxo-1,3-dihydro-isoindol-2-yl)-éthoxy]-4-isopropoxy-benzaldéhyde on obtient le composé du titre sous forme d'un solide blanc :
Rendement : 65%
F : 123°C
¹H RMN (CDCl₃) δ : 1.20 (d, 6H) ; 4.14 (t, 2H) ; 4.26 (t, 2H) ; 4.68 (s, 1H (échangeable)) ; 6.30 (dd, 1H) ; 6.46 (d, 1H) ; 6.74 (d, 1H) ; 7.73 (m, 2H) ; 7.85 (m, 2H) ;
IR (KBr) ν : 3252, 1711 et 1511 cm⁻¹.

### Exemple 11 : 2-[2-(2,5-Diisopropoxy-phénoxy)-éthyl]-isoindole-1,3-dione (4g)

En procédant comme l'exemple 7 mais en remplaçant le 2-[2-(5-fluoro-2-hydroxy-phénoxy)-éthyl]-isoindole-1,3-dione par le 2-[2-(5-hydroxy-2-isopropoxy-phénoxy)-éthyl)-isoindole-1,3-dione on obtient le composé du titre sous forme d'un solide blanc :
F : 66°C
¹H RMN (CDCl₃) δ : 1.20 (d, 6H) ; 1.28 (d, 6H) ; 4.12 (t, 2H) ; 4.22 (t, 2H) ; 4.26 (m, 1H) ; 4.42 (m, 1H) ; 6.35 (dd, 1H) ; 6.50 (d, 1H) ; 6.76 (d, 1H) ; 7.72 (m, 2H) ; 7.85 (m, 2H).

### Exemple 12 : [2-(5-Acetylamino-2-isopropoxy-phénoxy)-éthyl]-carbamic acid tert-butyl ester (4i)

Le composé du titre est obtenu sous forme d'une huile orangée :
Rendement : 80%
¹H RMN (CDCl₃) δ : 1.33 (d, 6H) ; 1.45 (s, 9H) ; 2.15 (s, 3H) ; 3.46 (dt, 2H) ; 4.04 (t, 2H) ; 4.43 (m, 1H) ; 5.28 (s, 1H) ; 6.96 (d, 1H) ; 7.06 (d, 1H) ; 7.27 (dd, 1H).

### Exemple 13 : 3-(bicyclo[2.2.1]hept-5-en-2-yloxy)-benzaldéhyde (2a-1)

On introduit à 0°C dans une solution de 3.66 g de 3-hydroxy-benzaldehyde (30 mmoles) dans du tétrahydrofurane (40 ml) 3 g de bicyclo [2.2.1]hept-5-en-2-ol (27.2 mmoles), puis 7.86 g de triphényphosphine (30 mmoles) et on ajoute goutte à goutte 13.6 ml d'azodicarboxylate d'éthyle (40% dans le toluène, 30 mmoles). Le mélange est agité à 75°C pendant 48 heures puis le solvant est évaporé, le résidu est repris dans du dichlorométhane et le mélange est lavé avec une solution aqueuse normale de soude, de l'eau, puis une solution aqueuse saturée de chlorure de sodium. On sèche sur sulfate de magnésium, on filtre et on concentre le mélange. Le produit du titre est isolé par chromatographie sur colonne de silice (éluant : cyclohexane / acétate d'éthyle, 88 / 12). On obtient 0.6 g d'une huile incolore :
Rendement : 10%
¹H RMN (DMSO d₆) δ : 1.34 (m, 1H) ; 1.42 (m, 1H) ; 1.53 (m, 1H) ; 1.74 (m, 1H) ; 2.89 (s, 1H) ; 2.99 (s, 1H) ; 4.38 (d, 1H) ; 6.10 (m, 1H) ; 6.35 (m, 1H) ; 7.28 (m, 1H) ; 7.40 (s, 1H) ; 7.46 (m, 2H) ; 9.97 (s, 1H).

### Exemple 14 : 2-méthoxy-5-cyclopenlyloxy-benzaldéhyde (2a-2)

On ajoute 5.45 g de carbonate de potassium (39.4 mmoles) et 4.2 ml de bromocyclopentane (39.4 mmoles) à une solution de 4 g de 5-hydroxy-2-méthoxybenzaldehyde (26.3 mmoles) dans 27 ml d'acétonitrile. Le mélange est agité pendant 16 heures à 80°C puis versé dans de l'eau glacée, extrait à l'éther et la phase organique est lavée avec une solution aqueuse normale de soude puis avec une solution aqueuse saturée de chlorure de sodium. La phase organique est séchée sur sulfate de magnésium, filtrée et le solvant est évaporé sous vide. Le produit du titre est isolé par distillation au four à boules (T = 200°C, P = 10 mbars). On obtient 4.13 g d'une huile jaune clair :
Rendement: 71 %
¹H RMN (CDCl₃) δ : 1.58- 1.63 (m, 2H) ; 1.83-2.06 (m, 6H) ; 3.89 (s, 3H) ; 4.73 (m, 1H) ; 6.94 (d, 1H) ; 7.11 (dd, 1H) ; 7.28 (d, 1H) ; 10.40 (s, 1H).

### Exemple 15 : 5-Cyclopentyloxy-2-fluoro-benzaldéhyde (2b-1)

### Etape 1 : 4-Cyclopentyloxy-1-fluoro-2-méthyl-benzène (8)

En procédant comme dans l'exemple 14, mais en remplaçant le 5-hydroxy-2-méthoxy-benzaldéhyde par le 4-fluoro-3-méthyl-phénol, on obtient le produit du titre sous forme d'une huile jaune pâle :
Rendement : 75%
¹H RMN (CDCl₃) δ : 1.60 (m, 2H) ; 1.77-1.90 (m, 6H) ; 2.22 (s, 3H) ; 4.68 (m, 1H) ; 6.60 (m, 1 H) ; 6.67 (m, 1H) ; 6.84 (t, 1H).

### Etape 2 : 2-Bromométhyl-4-cyclopentyloxy-1-fluoro-benzène (9)

On introduit 4.81 g de N-bromosuccinimide (27 mmoles) et 0.6 g de peroxyde de benzoyle (2 mmoles) dans une solution de 5 g de 4-cyclopentyloxy-1-fluoro-2-méthyl-benzène (25.8 mmoles) dans du tétrachlorure de carbone (130 ml). Le mélange est chauffé à 80°C pendant 17 heures puis on ajoute encore 2.29 g de N-bromosuccinimide (12.89 mmoles) et 0.3 g de peroxide de benzoyle ( 1 mmole) et on chauffe encore pendant 12 heures. Le mélange est ensuite refroidit à température ambiante et filtré sur celite. La liqueur mère est concentrée et l'huile noire obtenue est purifiée par chromatographie sur colonne de silice (éluant : cyclohexane / acétate d'éthyle, 88 / 12). On obtient 4.63 g d'une huile orange :
Rendement : 66%
¹H RMN (CDCl₃) δ : 1.60 (m, 2H) ; 1.79-1.86 (m, 6H) ; 4.66 (m, 2H) ; 4.68 (m, 1H) ; 6.69 (d, 1H) ; 6.89 (t, 1H) 7.18 (d, 1H).

### Etape 3 : 5-Cyclopentyloxy-2-fluoro-benzaldéhyde

On introduit 1.9 ml de s-collidine (14.3 mmoles) dans une solution de 3 g de 2-bromométhyl-4-cyclopentyloxy-1-fluoro-benzène (11 mmoles) dans 65 ml de diméthylsulfoxide. Le mélange est chauffé à 150°C pendant 25 minutes puis est refroidit à température ambiante. Le solvant est évaporé et le produit du titre est isolé par chromatographie sur colonne de silice (éluant : cyclohexane / acétate d'éthyle, 96 / 4). On obtient 1.1 g d'une huile jaune :
Rendement : 48%
¹H RMN (CDCl₃) δ : 1.61 (m, 2H) ; 1.68-1.72 (m, 4H) ; 1.88 (m, 2H) ; 4.33 (m , 1H) ; 7.24-7.32 (m, 3H) ; 10.16 (s, 1H).

### Exemple 16 : (3-Cyclopentyloxy-benzyl)-[2-(2-isopropoxy-phénoxy)-éthyl]-amine (1-1)

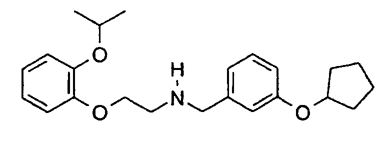

### Etape 1 : 2-(2-Isopropoxy-phénoxy)-éthylamine (3a-1)

On ajoute 1.10 g de 2-[2-(2-isopropoxy-phénoxy)-éthyl]-isoindole-1.3-dione (3.38 mmoles) à 4 ml d'éthanolamine (66.3 mmoles) puis on porte la solution à 60°C pendant 2 heures. Le mélange est versé dans l'eau glacée puis extrait à l'acétate d'éthyle. La phase organique est lavée avec une solution aqueuse saturée en chlorure de sodium, séchée sur sulfate de sodium, filtrée et le solvant évaporé sous vide. On obtient 0.66 g de produit du titre sous la forme d'une huile jaune pâle, utilisé directement dans l'étape suivante sans autre purification :
Rendement: 100%
¹H RMN (DMSO d₆) δ : 1.24 (d, 6H) ; 1.46 (s, 2H (échangeables)) ; 2.84 (t, 2H); 3.89 (t, 2H) ; 4.48 (m, 1H) ; 6.87 (m, 2H) ; 6.95 (m, 2H).

### Etape 2 : (3-Cyclopentyloxy-benzyl)-[2-(2-isopropoxy-phenoxy)-éthyl]-amine

On mélange 0.5 g de 2-(2-isopropoxy-phénoxy)-éthylamine (2.56 mmoles) et 0.49 de 3-cyclopentyloxy-benzaldehyde (2.56 mmoles) dans 20 ml de toluène. La solution est chauffé au reflux pendant 12 heures en éliminant l'eau formée en continue. Le toluène est alors évaporé, le résidu est repris dans 20 ml de méthanol et la solution est refroidie à 0°C. On ajoute par portions 0.28 g de borohydrure de potassium (5.12 mmoles) et on agite pendant 3 heures à température ambiante. Le méthanol est évaporé, le résidu est repris dans du dichlorométhane et la phase organique est lavée à l'eau puis avec une solution aqueuse saturée de chlorure de sodium. La phase organique est séchée sur sulfate de magnésium, filtrée et le solvant évaporé sous vide. On isole le produit du titre par chromatographie sur colonne de silice (éluant : dichloromethane / méthanol / ammoniaque, 98 / 1.5 / 0.5). On obtient 0.77 g du produit du titre sous forme d'une huile jaune pâle :
Rendement : 81%
¹H RMN (CDCl₃) δ : 1.31 (d, 6H) ; 1.60 (m, 2H) ; 1.77-1.92 (m, 4H) ; 2.07 (m, 2H) ; 3.03 (t, 2H) ; 3.85 (s, 2H) ; 4.13 (t, 2H) ; 4.47 (m, 1H) ; 4.76 (m, 1H) ; 6.75 (dd, 1H) ; 6.91 (m, 6H) ; 7.40 (t, 1H).

Préparation du sel : on dissous 0.77 g du produit du titre (2.08 mmoles) dans 10 ml d'éthanol puis on ajoute 0.18 g d'acide oxalique (2.08 mmoles) dans 10 ml d'éthanol. On concentre la solution, le sel précipite et on filtre la solution concentrée. Le sel est séché sous vide à 50°C. On obtient 0.83 g du composé du titre sous forme d'oxalate, poudre cristalline blanche :
F : 170°C

| Analyse C₂₅H₃₂NO₇ | | | |
|---|---|---|---|
| Calc % | C 65.34 | H 7.24 | N 3.05 |
| Tr. | 65.45 | 7.29 | 3.07 |

¹H RMN (DMSO d₆) δ : 1.24 (d, 6H) ; 1.58 (m, 2H) ; 1.69 (m, 4H) ; 1.92 (m, 2H) ; 3.26 (t, 2H) ; 4.24 (t, 2H) ; 4.26 (s, 2H) ; 4.55 (m, 1H) ; 4.81 (m, 1H) ; 6.87 - 6.97 (m, 3H) ; 7.01-7.10 (m, 4H) ; 7.32 (t, 1H) ;
IR (KBr) ν : 1612, 1686 et 2973 cm⁻¹.

Les composés de formule (1), obtenus à partir des intermédiaires ou d'intermédiaires analogues à ceux des exemples 1 à 15, selon un procédé similaire à celui de l'exemple 16 et comportant les substituants désirés sont rassemblés dans le tableau 1 ci-après.

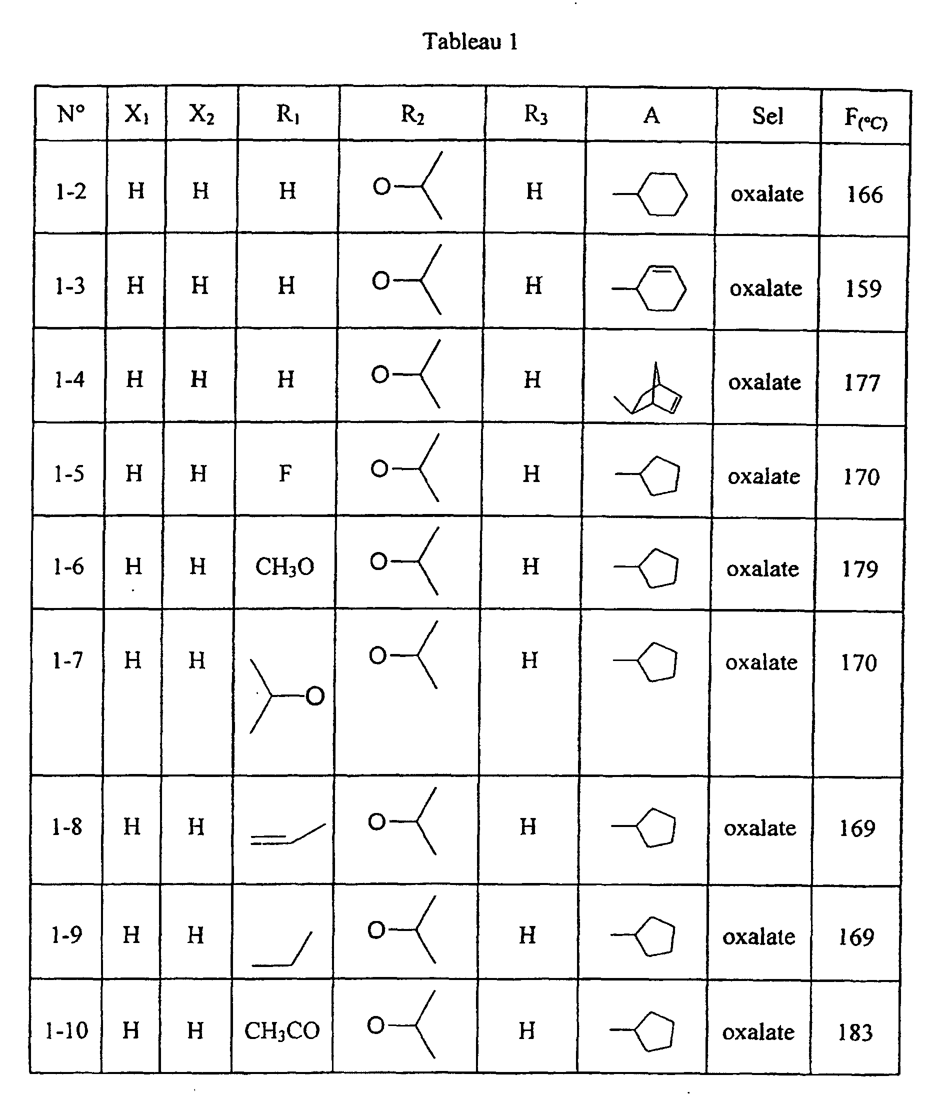

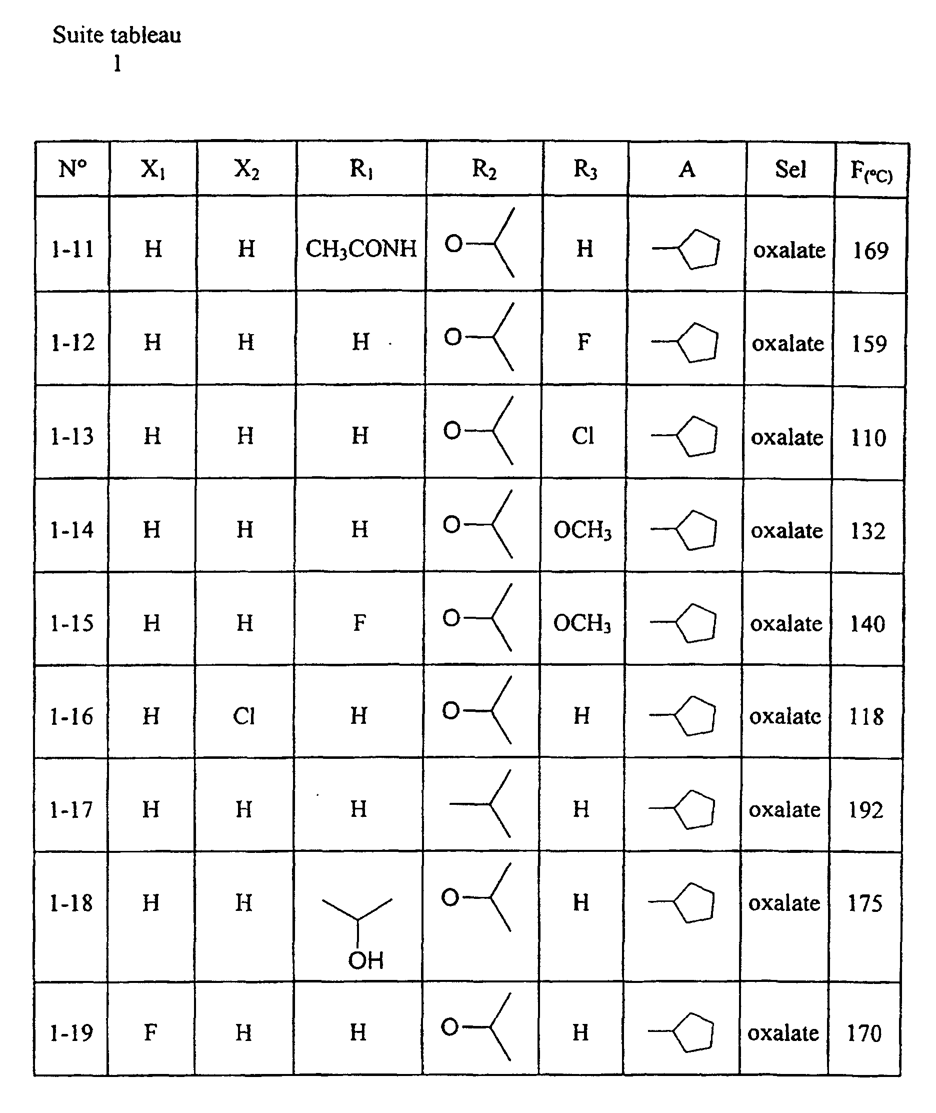

### Etude pharmacologique des composés de l'invention.

### 1- Mesure de l'affinité des composés de l'invention pour les récepteurs D₂.

L'affinité des composés de l'invention pour les récepteurs du type D₂ a été déterminé par la mesure du déplacement du (³H) YM-09151-2 (NET-1004 70-87 Ci / mmol), selon la méthode décrite dans Naunyn-Schimiedeberg's Arch. Pharmacol. Methods, 1985, 329, 333. Les valeurs de pKi (-log Ki) sont données sous forme de moyenne ± SEM d'au moins 3 expérimentations.

Le tableau 2 donne, à titre d'exemple, les pKi (D₂) de certains composés de l'invention en comparaison avec à la Risperidone.

### 2- Evaluation de l'activité antagoniste des récepteurs D₂ et des effets cataleptogènes des composés de l'invention in vivo.

Le test mettant en évidence l'activité antidopaminergique in vivo des composés de l'invention repose sur l'inhibition des comportements induit par le méthylphénidate, mesuré chez le rat, selon la méthode décrite dans J. Pharmacol. Exp. Ther. 1993, 267, 181.

Le test permettant d'évaluer la propension des produits de l'invention à provoquer des effets secondaires d'ordre extra-pyramidaux repose sur leur pouvoir cataleptogène, mesuré chez le rat, selon la méthode décrite dans Eur. J. Pharmacol. 1996, 313, 25.

A titre d'exemple, les valeurs obtenues après administration i.p. sont indiquées dans le tableau 2 en comparaison avec la substance de référence : la Risperidone.

**Tableau 2**

| **Composé** | **D**_{**2**} **pKi** | **normalisation** **ED**_{**50**} **mg / kg** | **Catalepsy** **ED**_{**50**} **mg / kg** |
|---|---|---|---|
| 1.4 | 8.56 | 5.0 | > 40 |
| 1.12 | 9.13 | 1.25 | > 40 |
| Risperidone | 8.70 | 2.9 | 4.6 |

Il ressort de cette étude que les composés de l'invention possèdent une haute affinité pour les récepteurs du type D₂ ainsi qu'une activité antidopaminergique puissante in vivo. Cependant, de façon surprenante, les composés de l'invention n'induisent pas ou n'induisent qu'à de très fortes doses des effets cataleptogènes comparativement avec la Risperidone. La Risperidone est un agent antipsychotique atypique utilisé en clinique (Inpharma® 1998, 1156, 5).

A ce titre, les composés de l'invention qui sont capables de moduler les effets de la dopamine endogène, sont utiles dans le traitement des désordres dopaminergiques tels que la schizophrénie, certaines maladies neurodégénératives et la dépendance à la cocaïne ou à l'alcool ou à des substances analogues.

## Revendications

1. Les composés de formule générale (1 ) 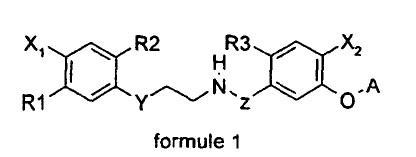 Dans laquelle :
X₁ est un atome d'hydrogène, de chlore ou de fluor ;
X₂ à la même signification que X₁ ;
R₁ représente :
- un atome d'hydrogène, de chlore, ou de fluor ;
- un substituant R₄, un groupe hydroxy (OH), un groupe alkoxy (OR₄), un groupe alkylcarbonyloxy (OC(O)R₄), un groupe alkylcarbonyl (C(O)R₄), un groupe amino (NH₂), un groupe alkylamino (NHR₄), un groupe dialkylamino (N(R₄)₂), un groupe (NHC(O)R₄) ou un groupe cyano (CN) ;
R₂ est un substituant R₄ ou un groupe alkoxy (OR₄) ;
R₃ à la même signification que R₁ ;
R₄ représente :
- un radical alkyle en C₁-C₅ linéaire ou ramifié, substitué ou non par 1 ou 2 atomes de fluor ou un groupe hydroxy (OH), contenant ou non une double liaison ;
- un radical cycloalkyle à 3, 4, 5 ou 6 chaînons, substitué ou non par 1 ou 2 atomes de fluor, contenant ou non une double liaison ;
Y est un atome d'oxygène, un groupe fluorométhylène (CHF) ou difluorométhylène (CF₂) ;
z est un groupe méthylène (CH₂), éventuellement substitué par 1 ou 2 groupes méthyle (CH₃) ou fluorométhyle (CH₂F) ;
A représente ;
- un radical cycloalkyle à 3, 4, 5 ou 6 chaînons ou un radical bicyclique à 7 ou 8 chaînons, contenant éventuellement une double liaison, une fonction oxo (=O), un groupe hydroxy (OH), un groupe méthoxy (OCH₃) ou 1 ou 2 atomes de fluor ;
- un groupe hétérocyclique non aromatique à 5 ou 6 chaînons contenant un ou deux hétéroatomes choisis parmi l'azote, l'oxygène, ou le soufre, éventuellement substitué par une fonction oxo (=O), un groupe hydroxy (OH), un groupe méthoxy (OCH₃) ou 1 ou 2 atomes d'halogène, ainsi que leurs sels d'addition avec des acides minéraux ou organiques pharmaceutiquement acceptables et les hydrates de ces sels, ces différents composés pouvant se présenter sous forme énantiomères purs, de mélanges énantiomères en toutes proportions y compris les mélanges racémiques.

2. Composé de formule générale (1) selon la revendication 1, **caractérisé en ce que** :
- R₂ représente un isopropoxy.

3. Composé de formule générale (1) selon l'une des revendications 1 et 2, **caractérisé en ce que** :
- Y représente un atome d'oxygène,
- Z représente un groupe méthylène.

4. Composé de formule générale (1) selon l'une des revendications 1 à 3, **caractérisé en ce que** :
- A est choisi parmi les groupes cyclopentyle, cyclohexyle, cyclohex-2-ényle, bicyclo[2.2.1]hept-5-en-2-yle.

5. Composés de formule générale (1), selon l'une des revendications 1 à 4, **caractérisés en ce qu'**ils sont choisis parmi :
(3-Cyclopentyloxy-benzyl)-[2-(2-isopropoxy-phenoxy)-éthyl]-amine
(3-Cyclohexyloxy- benzyl)-[2-(2-isopropoxy-phenoxy)-éthyl]-amine
[3-(Cyclohex-2-enyloxy)-benzyl]- [2-(2-isopropoxy-phenoxy)-éthyl]-amine
[3-(Bicyclo[2.2.1]hept-5-en-2-yloxy)-benzyl]- [2-(2-isopropoxy-phenoxy)-éthyl]-amine
(3-Cyclopentyloxy-benzyl)-[2-(5-fluoro-2-isopropoxy-phenoxy)-éthyl]-amine
(3-Cyclopentyloxy-benzyl)-[2-(2-isopropoxy-5-méthoxy-phenoxy)-éthyl]-amine
(3-Cyclopentyloxy-benzyl)-[2-(2,5-diisopropoxy-phenoxy)-éthyl]-amine
(3-Cyclopentyloxy-benzyl)-[2-(2-isopropoxy-5-vinyl-phenoxy)-éthyl]-amine
(3-Cyclopentyloxy-benzyl)-[2-(2-isopropoxy-5-éthyl-phenoxy)-éthyl]-amine
1- {3-[2-(3-Cyclopentyloxy-benzylamino)-éthoxy]-4-isopropoxy-phényl}-ethanone
N-{3-[2-(3-Cyclopentyloxy-benzylamino)-éthoxy]-4-isopropoxy-phényl}-acetamide
(5-Cyclopentyloxy-2-fluoro-benzyl)-[2-(2-isopropoxy-phenoxy)-éthyl]-amine
(2-Chloro-5-cyclopentyloxy-benzyl)-[2-(2-isopropoxy-phenoxy)-éthyl]-amine
(5-Cyclopentyloxy-2-méthoxy-benzyl)-(2-(2-isopropoxy-phenoxy)-éthyl]-amine
(5-Cyclopentyloxy-2-méthoxy-benzyl)-[2-(5-fluoro-2- isopropoxy-phenoxy)-éthyl]-amine
(4-Chloro-3-cyclopentyloxy-benzyl)-[2-(2-isopropoxy-phenoxy)-éthyl]-amine
(3-Cyclopentyloxy-benzyl)-[2-(4-fluoro-2-isopropoxy-phenoxy)-éthyl]-amine
(3-Cyclopentyloxy-benzyl)-[2-(2-isopropyl-phenoxy)-éthyl]-amine
1 {3-[2-(3-Cyclopentyloxy-benzylamino)-éthoxy]-4-isopropoxy-phényl}-ethanol.

6. A titre de médicament, les composés de formule (1) selon l'une des revendications 1 à 5.

7. Composition pharmaceutique **caractérisée en ce qu'**elle comprend au moins un composé de formule (1) selon l'une des revendications 1 à 5, ou un sel pharmaceutiquement acceptable d'un composé de formule (1) et un excipient approprié.

8. Utilisation d'un composé de formule (1) ou un sel pharmaceutiquement acceptable d'un composé de formule (1), selon l'une des revendications 1 à 5, en quantité suffisante pour la préparation d'un médicament destiné au traitement de la schizophrénie.

9. Utilisation d'un composé de formule (1), ou un sel pharmaceutiquement acceptable d'un composé de formule (1), selon l'une des revendications 1 à 5, en quantité suffisante pour la préparation d'un médicament destiné au traitement de la dépendance.

10. Utilisation d'un composé de formule (1) ou un sel pharmaceutiquement acceptable d'un composé de formule (1), selon l'une des revendications 1 à 5, en quantité suffisante pour la préparation d'un médicament destiné au traitement des maladies neurodégénératives.

## Claims

1. Compounds of general formula (1) 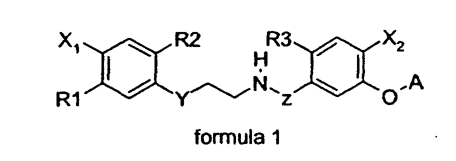 in which:
X₁ is a hydrogen, chlorine or fluorine atom;
X₂ has the same meaning as X₁;
R₁ represents:
- a hydrogen, chlorine or fluorine atom;
- a substituent R₄, a hydroxyl (OH) group, an alkoxy (OR₄) group, an alkylcarbonyloxy (OC(O)R₄) group, an alkylcarbonyl (C(O)R₄) group, an amino (NH₂) group, an alkylamino (NHR₄) group, a dialkylamino (N(R₄)₂) group, a (NHC(O)R₄) group or a cyano (CN) group;
R₂ is a substituent R₄ or an alkoxy (OR₄) group;
R₃ has the same meaning as R₁;
R₄ represents:
- a linear or branched C₁-C₅ alkyl radical optionally substituted with 1 or 2 fluorine atoms or a hydroxyl (OH) group and optionally containing a double bond;
- a 3-, 4-, 5- or 6-membered cycloalkyl radical optionally substituted with 1 or 2 fluorine atoms and optionally containing a double bond;
Y is an oxygen atom or a fluoromethylene (CHF) or difluoromethylene (CF₂) group;
z is a methylene (CH₂) group, optionally substituted with 1 or 2 methyl (CH₃) or fluoromethyl (CH₂F) groups;
A represents:
- a 3-, 4-, 5- or 6-membered cycloalkyl radical or a 7- or 8-membered bicyclic radical optionally containing a double bond, an oxo (=O) function, a hydroxyl (OH) group, a methoxy (OCH₃) group or 1 or 2 fluorine atoms;
- a 5- or 6-membered non-aromatic heterocyclic group containing one or two hetero atoms chosen from nitrogen, oxygen and sulfur, optionally substituted with an oxo (=O) function, a hydroxyl (OH) group, a methoxy (OCH₃) group or 1 or 2 halogen atoms, as well as the addition salts thereof with pharmaceutically acceptable mineral or organic acids and the hydrates of these salts, these various compounds possibly being in the form of pure enantiomers or enantiomeric mixtures in all proportions, including racemic mixtures.

2. Compound of general formula (1) according to Claim 1, **characterized in that**:
- R₂ represents an isopropoxy.

3. Compound of general formula (1) according to either of Claims 1 and 2, **characterized in that**:
- Y represents an oxygen atom,
- Z represents a methylene group.

4. Compound of general formula (1) according to one of Claims 1 to 3, **characterized in that**:
- A is chosen from cyclopentyl, cyclohexyl, 2-cyclohexenyl and bicyclo[2.2.1]hept-5-en-2-yl groups.

5. Compounds of general formula (1), according to one of Claims 1 to 4, **characterized in that** they are chosen from:
(3-Cyclopentyloxybenzyl)[2-(2-isopropoxyphenoxy)ethyl]amine
(3-Cyclohexyloxybenzyl)[2-(2-isopropoxyphenoxy)ethyl]amine
[3-(2- Cyclohexenyloxy)benzyl][2-(2-isopropoxyphenoxy)ethyl]amine
[3-(Bicyclo[2.2.1]hept-5-en-2-yloxy)benzyl][2-(2-isopropoxyphenoxy)ethyl]amine
(3-Cyclopentyloxybenzyl)[2-(5-fluoro-2-isopropoxyphenoxy)ethyl]amine
(3-Cyclopentyloxybenzyl)[2-(2-isopropoxy-5-methoxyphenoxy)ethyl]amine
(3-Cyclopentyloxybenzyl)[2-(2,5-diisopropoxyphenoxy)ethyl]amine
(3-Cyclopentyloxybenzyl)[2-(2-isopropoxy-5-vinylphenoxy)ethyl]amine
(3-Cyclopentyloxybenzyl)[2-(2-isopropoxy-5-ethylphenoxy)ethyl]amine
1-{3-[2-(3-Cyclopentyloxybenzylamino)ethoxy]-4-isopropoxyphenyl}ethanone
N- {3-[2-(3-Cyclopentyloxybenzylamino)ethoxy]-4-isopropoxyphenyl}acetamide
(5-Cyclopentyloxy-2-fluorobenzyl)[2-(2-isopropoxyphenoxy)ethyl]amine
(2-Chloro-5-cyclopentyloxybenzyl)[2-(2-isopropoxyphenoxy)ethyl]amine
(5-Cyclopentyloxy-2-methoxybenzyl)[2-(2-isopropoxyphenoxy)ethyl]amine
(5-Cyclopentyloxy-2-methoxybenzyl)[2-(5-fluoro-2-isopropoxyphenoxy)ethyl]amine
(4-Chloro-3-cyclopentyloxybenzyl)[2-(2-isopropoxyphenoxy)ethyl]amine
(3-Cyclopentyloxybenzyl)[2-(4-fluoro-2-isopropoxyphenoxy)ethyl]amine
(3-Cyclopentyloxybenzyl)[2-(2-isopropylphenoxy)ethyl]amine
1 {3-[2-(3-Cyclopentyloxybenzylamino)ethoxy]-4-isopropoxyphenyl}ethanol.

6. As a medicinal product, the compounds of formula (1) according to one of Claims 1 to 5.

7. Pharmaceutical composition, **characterized in that** it comprises at least one compound of formula (1) according to one of Claims 1 to 5, or a pharmaceutically acceptable salt of a compound of formula (1) and a suitable excipient.

8. Use of a compound of formula (1) or a pharmaceutically acceptable salt of a compound of formula (1), according to one of Claims 1 to 5, in an amount which is sufficient to prepare a medicinal product for treating schizophrenia.

9. Use of a compound of formula (1) or a pharmaceutically acceptable salt of a compound of formula (1), according to one of Claims 1 to 5, in an amount which is sufficient to prepare a medicinal product for treating dependency.

10. Use of a compound of formula (1) or a pharmaceutically acceptable salt of a compound of formula (1), according to one of Claims 1 to 5, in an amount which is sufficient to prepare a medicinal product for treating neurodegenerative diseases.

## Patentansprüche

1. Verbindungen der allgemeinen Formel (1) 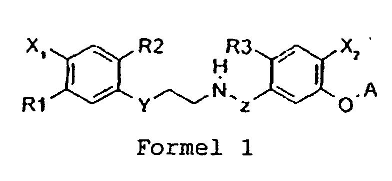 in welcher:
X₁ ein Wasserstoff-, Chlor- oder Fluoratom ist;
X₂ die gleiche Bedeutung wie X₁ hat;
R₁ steht für:
- ein Wasserstoff-, Chlor- oder Fluoratom;
- einen Substituenten R₄, eine Hydroxygruppe (OH), eine Alkoxygruppe (OR₄), eine Alkylcarbonyloxygruppe (OC(O)R₄), eine Alkylcarbonylgruppe (C(O)R₄), eine Aminogruppe (NH₂), eine Alkylaminogruppe (NHR₄), eine Dialkylaminogruppe (N(R₄)₂), eine Gruppe (NHC(O)R₄) oder eine Cyanogruppe (CN);
R₂ ein Substituent R₄ oder eine Alkoxygruppe (OR₄) ist;
R₃ die gleiche Bedeutung wie R₁ hat;
R₄ steht für:
- einen linearen oder verzweigten C₁-C₅-Rest, welcher durch 1 oder 2 Fluoratome oder eine Hydroxygruppe (OH) substituiert oder nicht substituiert ist, welcher eine Doppelbindung enthält oder nicht;
- einen Cycloalkylrest mit 3, 4, 5 oder 6 Kettengliedern, welcher durch 1 oder 2 Fluoratome substituiert oder nicht substituiert ist, welcher eine Doppelbindung enthält oder nicht;
Y ein Sauerstoffatom, eine Fluormethylengruppe (CHF) oder Difluormethylengruppe (CF₂) ist;
z eine Methylengruppe (CH₂), welche gegebenenfalls durch 1 oder 2 Methylgruppen (CH₃) oder Fluormethylgruppen (CH₂F) substituiert ist, ist;
A steht für:
- einen Cycloalkylrest mit 3, 4, 5 oder 6 Kettengliedern oder einen bicyclischen Rest mit 7 oder 8 Kettengliedern, welcher gegebenenfalls eine Doppelbindung, eine Oxofunktion (=O), eine Hydroxygruppe (OH), eine Methoxygruppe (OCH₃) oder 1 oder 2 Fluoratome enthält;
- eine nicht-aromatische heterocyclische Gruppe mit 5 oder 6 Kettengliedern, welche ein oder zwei Heteroatome, ausgewählt unter Stickstoff, Sauerstoff oder Schwefel, enthält, welche gegebenenfalls durch eine Oxofunktion (=O), eine Hydroxygruppe (OH), eine Methoxygruppe (OCH₃) oder 1 oder 2 Halogenatome substituiert ist,
sowie deren Additionssalze mit pharmazeutisch annehmbaren anorganischen oder organischen Säuren und die Hydrate dieser Salze, wobei diese verschiedenen Verbindungen in Form von reinen Enantiomeren, von Enantiomerenmischungen in allen Verhältnissen, einschließlich racemischer Mischungen, vorliegen können.

2. Verbindung der allgemeinen Formel (1) nach Anspruch 1, **dadurch gekennzeichnet, dass**:
- R₂ für ein Isopropoxy steht.

3. Verbindung der allgemeinen Formel (1) nach einem der Ansprüche 1 und 2, **dadurch gekennzeichnet, dass**:
- Y für ein Sauerstoffatom steht;
- Z für eine Methylengruppe steht.

4. Verbindung der allgemeinen Formel (1) nach einem der Ansprüche 1 bis 3, **dadurch gekennzeichnet, dass**:
- A aus den Cyclopentyl-, Cyclohexyl-, Cyclohex-2-enyl-, Bicyclo[2.2.1]hept-5-en-2-ylgruppen ausgewählt wird.

5. Verbindungen der allgemeinen Formel (1) nach einem der Ansprüche 1 bis 4, **dadurch gekennzeichnet, dass** sie ausgewählt werden unter:
(3-Cyclopentyloxybenzyl)-[2-(2-isopropoxyphenoxy)ethyl]amin
(3-Cyclohexyloxybenzyl)-[2-(2-isopropoxyphenoxy)ethyl]amin
[3-(Cyclohex-2-enyloxy)benzyl]-[2-(2-isopropoxyphenoxy)ethyl]-amin
[3-(Bicyclo[2.2.1]hept-5-en-2-yloxy)benzyl]-[2-(2-isopropoxyphenoxy)ethyl]amin
(3-Cyclopentyloxybenzyl)-[2-(5-fluor-2-isopropoxyphenoxy)-ethyl]amin
(3-Cyclopentyloxybenzyl)-[2-(2-isopropoxy-5-methoxy-phenoxy)-ethyl]amin
(3-Cyclopentyloxybenzyl)-[2-(2,5-diisopropoxyphenoxy)ethyl]-amin
(3-Cyclopentyloxybenzyl)-[2-(2-isopropoxy-5-vinyl-phenoxy)-ethyl]amin
(3-Cyclopentyloxybenzyl)-[2-(2-isopropoxy-5-ethyl-phenoxy)-ethyl]amin
1-{3-[2-(3-Cyclopentyloxybenzylamino)ethoxy]-4-isopropoxyphenyl}ethanon
N-{3-[2-(3-Cyclopentyloxybenzylamino)ethoxy]-4-isopropoxyphenyl}acetamid
(5-Cyclopentyloxy-2-fluorbenzyl)-[2-(2-isopropoxyphenoxy)-ethyl]amin
(2-Chlor-5-cyclopentyloxybenzyl)-[2-(2-isopropoxyphenoxy)-ethyl]amin
(5-Cyclopentyloxy-2-methoxybenzyl)-[2-(2-isopropoxyphenoxy)-ethyl]amin
(5-Cyclopentyloxy-2-methoxybenzyl)-[2-(5-fluor-2-isopropoxyphenoxy)ethyl]amin
(4-Chlor-3-cyclopentyloxybenzyl)-[2-(2-isopropoxyphenoxy)-ethyl]amin
(3-Cyclopentyloxybenzyl)-[2-(4-fluor-2-isopropoxyphenoxy)-ethyl]amin
(3-Cyclopentyloxybenzyl)-[2-(2-isopropylphenoxy)ethyl]amin
1-{3-[2-(3-Cyclopentyloxybenzylamino)ethoxy]-4-isopropoxyphenyl}ethanol.

6. Verbindungen der Formel (1) nach einem der Ansprüche 1 bis 5 als Arzneimittel.

7. Pharmazeutische Zusammensetzung, **dadurch gekennzeichnet, dass** sie wenigstens eine Verbindung der Formel (1) nach einem der Ansprüche 1 bis 5 oder ein pharmazeutisch annehmbares Salz einer Verbindung der Formel (1) und einen geeigneten Träger umfasst.

8. Verwendung einer Verbindung der Formel (1) oder eines pharmazeutisch annehmbaren Salzes einer Verbindung der Formel (1) nach einem der Ansprüche 1 bis 5 in ausreichender Menge für die Herstellung eines Arzneimittels, welches zur Behandlung von Schizophrenie bestimmt ist.

9. Verwendung einer Verbindung der Formel (1) oder eines pharmazeutisch annehmbaren Salzes einer Verbindung der Formel (1) nach einem der Ansprüche 1 bis 5 in ausreichender Menge für die Herstellung eines Arzneimittels, welches zur Behandlung von Abhängigkeit bestimmt ist.

10. Verwendung einer Verbindung der Formel (1) oder eines pharmazeutisch annehmbaren Salzes einer Verbindung der Formel (1) nach einem der Ansprüche 1 bis 5 in ausreichender Menge für die Herstellung eines Arzneimittels, welches zur Behandlung von neurodegenerativen Erkrankungen bestimmt ist.
